# EUROPÄISCHE PATENTSCHRIFT

(11) **EP 0 326 097 B1**
(45) Veröffentlichungstag und Bekanntmachung des Hinweises auf die Patenterteilung: **13.12.1995**
(21) Anmeldenummer: 89101259.3
(22) Anmeldetag: 25.01.1989
(51) Int. Cl.: A61C 13/00, A61K 6/00, B05D 3/12, C09J 5/02, B24C 11/00

(54) **Verfahren zur Vorbereitung einer Substratoberfläche für die Verbindung mit Kunststoff durch Aufbringen einer Schicht und Verwendung von gegebenenfalls silanisiertem und/oder von silanisiertem, siliciumhaltigem Material**
Process for pretreating a substrate surface for bonding with plastic by applying a layer and use of a possibly silanated and/or silanated silicon-containing compound
Procédé pour prétraiter la surface d'un substrat en vue de sa liaison avec une matière plastique par application d'une couche et utilisation d'un composé éventuellement silané et/ou d'un composé de silicium silané

(30) Priorität: 25.01.1988 DE 3802042
(43) Veröffentlichungstag der Anmeldung: 02.08.1989
(73) Patentinhaber: THERA Patent GmbH & Co. KG Gesellschaft für industrielle Schutzrechte, D-82229 Seefeld (DE)
(72) Erfinder: Gasser, Oswald, Dr., D-8031 Seefeld 1 (DE); Guggenberger, Rainer, Dr., D-8031 Seefeld 2 (DE); Burger, Bernd, D-8031 Alling (DE)
(74) Vertreter: Abitz, Walter, Dr.-Ing.

(56) Entgegenhaltungen:
- EP-A- 0 270 900
- DE-A- 3 211 123
- DE-C- 3 802 043
- US-A- 3 541 017
- US-A- 4 364 731

## Beschreibung

Der Erfindung betrifft ein Verfahren zur Vorbereitung einer Substratoberfläche für die Verbindung der Substratoberfläche mit Kunststoff, z.B. mit einem kunststoffhaltigen Kleber.

Klebeverbindungen zwischen Substratoberflächen, z.B. aus Holz, Gläsern, Metallen, Keramik oder Kunststoffen, mittels kunststoffhaltiger Kleber oder Beschichtungen von Substraten mit Kunststoffen sollen auch unter den unterschiedlichsten Witterungsbedingungen dauerhaft sein. Die Adhäsionsfestigkeit der Substrat-Kunststoff-Verbindung sollte im Idealfall bei den unterschiedlichsten Belastungen, wie mechanischen oder thermischen Verformungen, stets fester sein als die innere Festigkeit des Kunststoffs (Kohäsionsfestigkeit).

Bei der Herstellung von kunststoffverkleideten Kronenverblendungen auf einem metallischen Zahnersatz ist es besonders wichtig, die Kunststoffe mit den Metallen spaltfrei und dauerhaft zu verbinden. Ein mangelhafter Verbund führt zum vorzeitigen Abplatzen der Verblendung, zur Randspaltbildung gegebenenfalls mit Verfärbung des Randes durch Oxidation des Metallgerüsts und zu mechanischer Gewebsirritation am Spalt zwischen Verblendung und Metallgerüst.

Im Mundmilieu unterliegt der Metall-Kunststoff-Verbund oder Keramik-Kunststoff-Verbund besonderen Belastungen. Dies sind zum einen physikalischmechanische Belastungen, wie sie bei den Kaubewegungen auftreten, sowie chemisch-biologische Belastungen durch Speichel-, Nahrungsmittel- und Medikamenteneinfluss. Durch die im Mundmilieu zudem auftretenden Temperaturwechsel ist der Verbund weiteren Stresswirkungen ausgesetzt.

Bisherige Lösungswege führen entweder zu nicht genügend dauerhaften Verbindungen oder erfordern einen hohen, in zahntechnischen Labors normalerweise nicht akzeptablen, apparativen Aufwand und zudem eine exzellente Beherrschung des Verfahrens zur Erlangung eines optimalen Verbundes.

So ist aus der DE-A 32 11 123 ein Verfahren zum Aufbringen einer Kronenverblendung auf einen metallischen Zahnersatz bekannt, bei welchem der metallische Kronenkörper durch Sandstrahlung aufgerauht, dann mehrmals in ein Ultraschallbad, das Silan enthält, eingetaucht und hierauf getrocknet wird. Anschliessend wird in bekannter Weise der Verblendkunststoff aufgebracht. Nachteile der beschriebenen Arbeitsweise sind, dass lediglich siliciumhaltige Nicht-Edelmetall-Legierungen verwendet werden können und die erzielten Haftwerte einer Dauerbelastung im Mund nicht standhalten.

Die US-A 4 364 731 beschreibt und Herstellung einer Haftvermittlerschicht aus anorganischen Oxiden, beispielsweise Siliciumdioxid, die mittels einer sogenannten Sputter-Vorrichtung auf die Metalloberfläche aufgebracht werden. Die erhaltene Oxidschicht wird silanisiert und anschliessend das Verblendmaterial in bekannter Weise aufgebracht. Der Sputter-Prozess bringt es mit sich, dass die zu beschichtende Metalloberfläche sehr hohen Temperaturen ausgesetzt ist, zudem ist das Verfahren nur mit enormem Kostenaufwand in den Labors zu verwirklichen.

Eine Weiterentwicklung zu oben genannter US-Patentschrift findet sich in der europäischen Patentveröffentlichungsschrift 0 151 233. Hierbei wird die siliciumoxidhaltige Haftvermittlerschicht mit einem Flammhydrolysebrenner erzeugt.

Diese Haftvermittlerschicht wird silanisiert und anschliessend in bekannter Weise der Verblendkunststoff aufgetragen. Auch bei diesem Prozess wird das Werkstück relativ hohen Temperaturen ausgesetzt, gute Haftwerte werden nur unter genauester Einhaltung aller Geräteparameter erhalten, was nur mittels sehr hohem apparativem Aufwand zu erreichen ist.

Weiterhin ist aus der DE-A 36 42 290 ein Verfahren zur Verbesserung der Haftung von Kunststoffen auf Metallen bekannt, bei dem auf die Metalloberfläche eine Siliciumdioxidschicht aufgebracht wird, indem Kieselsole oder feine Dispersionen von feinstteiliger Kieselsäure auf die Metalloberfläche aufgebracht werden und die so erhaltenen Schichten bei Temperaturen von 100 bis 800°C eingebrannt werden. Auch bei diesem Verfahren ist das Werkstück hohen Temperaturen ausgesetzt und die damit erreichten Haftwerte, insbesondere bei Verwendung von Edelmetallen, die in der Zahntechnik sehr gebräuchlich sind, sind für eine dauerhafte Restauration im Mund nicht ausreichend.

Übliches Sandstrahlen mit Siliciumdioxid (E. C. Combe, "Zahnärztliche Werkstoffe", Carl Hanser Verlag München-Wien, S. 299, 1984; DE-A-35 31 892 und "Metalloberfläche", Bd. 37, S. 335, 1983) oder Aluminiumoxid (Derwent Abstr., 84-228034/37 - durchschnittliche Korngrösse 20-60 »m und US-A-45 04 228 - Korngrösse ca. 150 »m) oder die Oberflächenbehandlung durch Schleuderstrahlanlagen mit Stahlkies (A.W. Mallory, Industrie-Lackier-Betrieb, S. 223, 1985) führen nur zu einer Reinigung und Aufrauhung der Metalloberfläche. Zwar kann es auch zu vereinzelten Einschlüssen des Strahlgutes in die Oberfläche kommen (K.-A. van Oeteren, "Korrosionsschutz durch Anstrichstoffe", Bd. 1, S. 328, 1980), eine haftvermittelnde Schicht wird jedoch durch keines dieser Verfahren gewonnen.

Die Aufgabe der Erfindung besteht demzufolge darin, eine Substratoberfläche durch Aufbringen einer Schicht so vorzubereiten, dass die Herstellung einer dauerhaften Verbindung zwischen Kunststoff und Substratoberfläche möglich ist, wobei das Verfahren leicht und ohne grossen apparativen Aufwand durchführbar sein soll, das Werkstück nicht übermässig thermisch belastet werden soll und das Verfahren zu einer Substrat-Kunststoff-Verbindung führen soll, die gegenüber physikalischen, thermischen sowie hydrolytischen Wechsellasten beständig ist.

Diese Aufgabe wird erfindungsgemäss durch ein Verfahren gelöst, bei welchem man auf die Substratoberfläche eine Schicht durch Sandstrahlen mit einem Mittel aufbringt, das, bezogen auf das Gewicht des gesamten Sandstrahlmittels, aus
(A) 0,01 bis 90 Gew.-% gegebenenfalls silanisiertem Material mit einer Korngrösse < 5 »m und einer grösseren Härte als die der Substratoberfläche und/oder
(B) 20 bis 100 Gew.-% silanisiertem, siliciumhaltigem Material mit einer mittleren Korngrösse von 2 bis 200 »m und zum
(C) Rest aus einem Sandstrahlmittel mit einer mittleren Korngrösse > 5 »m
besteht und gegebenenfalls die so erhaltene Haftvermittlerschicht anschliessend silanisiert.

Mit "Korngrösse" ist die Primärpartikelgrösse gemeint.

Die Härte des Substrats und der Komponente (A) kann nach den Methoden von Mohs, Brinell, Knoop oder Vickers bestimmt werden, wobei für die Härteprüfung von Substrat und (A) jeweils die gleiche Methode verwendet werden soll. Bei der Härteprüfung von sehr feinem Material (< 1 »m) verwendet man das gleiche Material in gröberer Form. Mit der erfindungsgemässen Verwendung der Komponente (A), deren Härte grösser ist als die Härte des Substrats, wird eine um mindestens 30 %, vorzugsweise 50 % erhöhte Haftfestigkeit erzielt, als beim Sandstrahlen ohne die Komponente (A).

Die erfindungsgemäss vorbereiteten Substratoberflächen können mit im Handel erhältlichen Kleb-, Beschichtungs- oder Verblendmassen verklebt, beschichtet oder verblendet werden. Man kann so Holz-, Metall-, Keramik-, Kunststoff- oder Glasflächen mit anderen Holz-, Metall-, Glas-, Keramik- oder Kunststoffflächen verkleben.

Das erfindungsgemässe Verfahren weist folgende Vorteile auf:
- Das Verfahren benötigt weder eine Sputter-Vorrichtung, noch einen Flammhydrolysebrenner, noch einen Keramikofen zum Aufbringen der siliciumhaltigen Haftvermittlerschicht, sondern nur eine leicht verfügbare Sandstrahleinrichtung.
- Beim erfindungsgemässen Verfahren können die handelsüblichen Edel- sowie Nichtedelmetalle und deren Legierungen verwendet werden.
- Bei dem Verfahren treten keine hohen Temperaturen auf, bei welchen immer die Gefahr besteht, dass sich das metallische Werkstück verzieht.
- Ein besonderer Vorteil ist es, dass das erfindungsgemässe Verfahren auch zur Reparatur von beschädigten Teilen in situ, z.B. von im Mund befindlichen Kronen und Brücken mit Metallgerüsten, eingesetzt werden kann. Dies ist mit den geschilderten Verfahren des Standes der Technik kaum möglich.
- Ein weiterer besonderer Vorteil ist, dass der nach der erfindungsgemässen Vorbehandlung der Substratoberfläche hergestellte Substrat-Kunststoff-Verbund über eine ausgezeichnete hydrolytische Stabilität verfügt. Die Klebeverbindung besitzt auch nach längerer Lagerzeit in Wasser oder im Mundmilieu eine hohe Lagerfestigkeit.

Beim erfindungsgemässen Verfahren können Holz-, Metall-, Keramik-, Glas- oder Kunststoffoberflächen eingesetzt werden, bevorzugt werden Metalloberflächen eingesetzt.

Als Komponente (A) werden eingesetzt 0,01 bis 90 Gew.-%, bezogen auf das Gewicht des gesamten Sandstrahlmittels, gegebenenfalls silanisiertes Material mit einer Korngrösse < 5 »m und einer grösseren Härte als die der Substratoberfläche, bevorzugt 0,1 bis 30 Gew.-%, insbesondere eines Materials mit einer Korngrösse < 1 »m. Besonders bevorzugt ist Material (A) mit einer Korngrösse < 0,1 »m.

In einer bevorzugten Ausführungsform ist das als Komponente (A) verwendete Material silanisiert.

Vorzugsweise setzt man als Komponente (A) siliciumhaltiges Material ein, ganz besonders 0,01 - 50 Gew.-% gegebenenfalls silanisiertes, siliciumhaltiges Material.

Besonders bevorzugt als Komponente (A) sind: Quarz, Quarzglas, Silikatglas mit mindestens 10 Gew.-% Silicium, Siliciumcarbid, Siliciumnitrid und/oder pyrogene Kieselsäure; Aluminiumoxid, Titandioxid und/oder Zirkondioxid sowie die Oxide, Nitride und/oder Carbide der dritten und vierten Hauptgruppe sowie der Nebengruppe 4b. Dieses Material kann beispielsweise eine mittlere Korngrösse von 10 »m besitzen, es muss jedoch noch mindestens 0,01 Gew.-% Anteil an gegebenenfalls silanisiertem Material < 5 »m enthalten. Ganz besonders bevorzugt ist pyrogene Kieselsäure mit einer mittleren Korngrösse von 0,001 bis 0,05 »m.

Die Komponente (A) muss unter den Reaktionsbedingungen inert sein. Bevorzugte Komponenten (A) sind auch bei Aufschlagstemperaturen >1000°C inert.

Als Komponente (B) finden Verwendung 20 bis 100 Gew.-%, bezogen auf das Gewicht des gesamten Sandstrahlmittels, silanisiertes, siliciumhaltiges Material mit einer mittleren Korngrösse von 2 bis 200 »m; bevorzugt verwendet werden können 50 bis 100 Gew.-% mit einer bevorzugten mittleren Korngrösse von 5 bis 100 »m. Als silanisiertes siliciumhaltiges Material verwendet werden können beispielsweise Quarz, Quarzglas, Silikatglas mit mindestens 10 Gew.-% Silicium, Siliciumnitrid, Siliciumcarbid oder keramisches Material mit mindestens 10 Gew.-% Silicium. Besonders bevorzugt werden Quarzglas, Silikatglas sowie amorphes Siliciumnitrid. Bei der Verwendung von Silikatgläsern und siliciumhaltigem keramischem Material werden Materialien mit einem Siliciumgehalt > 30 Gew.-% bevorzugt.

Das erfindungsgemäss als (C) zu verwendende Sandstrahlmittel mit einer mittleren Korngrösse > 5 »m ist beispielsweise Aluminiumoxid (Korund). Die mittlere Korngrösse beträgt vorzugsweise > 5 bis 500 »m, insbesondere 20 bis 250 »m. Vorteilhaft können aber auch Quarz, Quarzglas, Silikate, Silikatgläser, Siliciumnitrid, Siliciumcarbid oder keramisches Material mit einem Siliciumgehalt verwendet werden.

Vorzugsweise verwendet man als Komponenten (A) und/oder (B) und (C) ein Material gleicher Zusammensetzung, aber unterschiedlicher Korngrösse.

Wenn Bestandteile des Sandstrahlmittels silanisiert sind, so enthalten sie vorzugsweise 0,1 bis 20 Gew.-% Silan, besonders bevorzugt 1 bis 5 Gew.-%, bezogen auf das Gewicht des Bestandteils des Sandstrahlmittels. Die Silanisierung erfolgt in an sich bekannter Weise, wie sie z.B. in der Füllstofftechnologie verwendet wird. Alle üblicherweise eingesetzten Silane eignen sich zum Herstellen der erfindungsgemäss verwendeten Bestandteile des Sandstrahlmittels, besonders geeignet sind Vinyltrimethoxysilan, γ-Glycidoxypropyltrimethoxysilan, γ-Methacroyloxypropyltrimethoxysilan und Tetramethyldivinyl-silazan. Die Verbindungen werden vorzugsweise in Form alkoholischer oder wässrigsaurer - z.B. essigsaurer - Lösung verwendet.

Die Silanisierung der aufgebrachten siliciumhaltigen Haftvermittlungsschicht erfolgt in an sich bekannter Weise. Bevorzugt verwendete Silane sind Vinyltrimethoxysilan, γ-Glycidoxypropyltrimethoxysilan, γ-Methacroyloxypropyltrimethoxysilan und Tetramethyl-divinyl-silazan. Die Verbindungen werden vorzugsweise in Form alkoholischer oder wässrigsaurer - z.B. essigsaurer - Lösung verwendet.

Es hat sich als besonders vorteilhaft erwiesen, sowohl bei der Silanisierung von Bestandteilen des Strahlmittels als auch bei der Silanisierung der erfindungsgemäss behandelten Oberfläche des Metalls jeweils Silane mit den gleichen funktionellen Gruppen einzusetzen, wie sie auch im Monomeren des Kunststoffs, z.B. des Klebers, vorhanden sind. So setzt man zum Verkleben von Metallen mit Epoxyharzen beispielsweise vorteilhaft Silane mit Epoxyendgruppen ein.

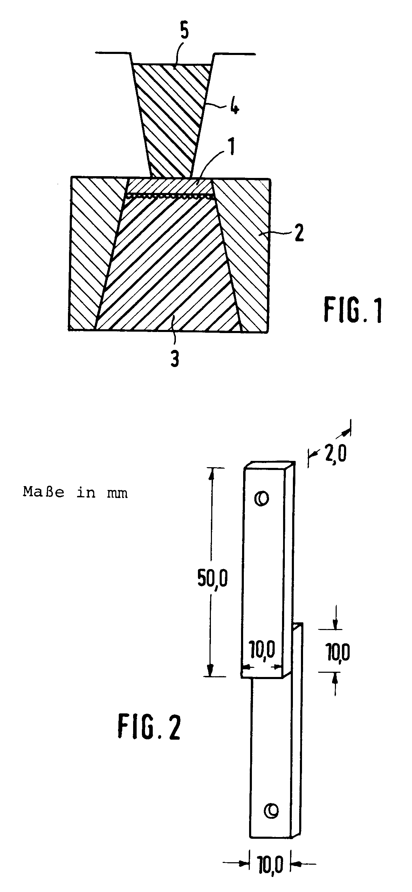

Zur weiteren Erläuterung der Erfindung dienen die im folgenden aufgeführten Beispiele, in welchen anhand von Modellversuchen gezeigt wird, dass das erfindungsgemässe Verfahren zu einer überraschend hohen Haftfestigkeit und Dauerhaftigkeit des Verbundes zwischen Metallen und Kunststoffen führt.
a) BEISPIELE FÜR KUNSTSTOFFBESCHICHTUNGEN VON DENTALEN METALLEGIERUNGEN
   Messverfahren
   Zur Überprüfung der Haftfestigkeit von Kunststoff an Metall wurde folgender Zugversuch durchgeführt (siehe Figur 1):
   Von den zu prüfenden Metallen wurden runde Plättchen (1) gegossen mit Durchmesser 12 mm, Dicke 2 mm, an der Rückseite mit Retentionsperlen versehen. Die Prüffläche wurde zur Reinigung 5 sec. mit Aluminiumoxid 250 »m abgestrahlt.
   Nach Durchführung der Oberflächenbehandlung wurde ein Opaker auf Methacrylat-Basis (Dentacolor-Opaker, Fa. Kulzer) aufgetragen. Nach dessen Aushärtung (90 sec. im Dentacolor XS-Gerät, Fa. Kulzer) wurde das Prüfplättchen (1) in der Halterung (2) durch Ausgiessen mit selbsthärtendem Kunststoff (Vitron, Fa. ESPE) (3) befestigt. Danach wurde ein rundes, nach oben sich konisch öffnendes Metallhütchen (4) (Durchmesser an der Haftfläche 7 mm) aufgesetzt und schichtweise mit lichthärtendem Verblendkunststoff (5) (Visio® -Gem, Fa. ESPE) aufgefüllt und ausgehärtet.
   Die so präparierten Proben wurden 20 h bei 36°C in Wasser gelagert, danach 6 h einem Temperaturwechselbad (15°C ↔ 70°C) mit einminütigen Wechseln ausgesetzt. Anschliessend wurden die Proben in eine Universalprüfmaschine (Fa. Zwick, Modell 1435) eingespannt und mit einem Vorschub von 1 mm/min. auseinandergezogen. Die Abrisskraft wurde gemessen und daraus die Haftfestigkeit im MPa berechnet. Pro Versuch wurden fünf Prüfplättchen eingesetzt und daraus der Mittelwert gebildet.

### Vergleichsbeispiele 1 - 4

Haftwerte auf Edelmetall-Legierung: Degulor M, Fa. Degussa

| Nr. | Verfahren | Nachbehandlung (Silanisierung) | Haftfestigkeit (MPa) Durchschnitt aus 5 Proben |
|---|---|---|---|
| 1 | mechanische Perlretentionen, Durchmesser 0,6 mm | --- | 8,8 |
| 2 | sandstrahlen⁽¹⁾ mit Al₂O₃, (mittlere Korngrösse ca. 250 »m) | --- | 2,9 |
| 3 | sandstrahlen⁽¹⁾ mit Al₂O₃, (mittlere Korngrösse ca. 250 »m) | Silanisierlösung A⁽²⁾ | 3,7 |
| 4 | sandstrahlen⁽¹⁾ mit unsilanisiertem Quarz (mittlere Korngrösse 25 »m; Anteil mit Korngrösse < 5 »m weniger als 0,01 Gew.-%) | Silanisierlösung A⁽²⁾ | 2,5 |

| | | | |
|---|---|---|---|
| (1) Zeit: 10 sec. Druck: 4 bar, Sandstrahlgerät FG 3-82 Sandmaster, Fa. Unitol AG/LTD., CH-4663 Aarburg | | | |
| (2) Silanisierlösung A = frisch bereitete Lösung aus 1 Teil γ-Methacroyloxy-propyl-trimethoxysilan und 15 Teilen einer Lösung aus 87 % Ethanol, 3 % Essigsäure und 10 % Wasser | | | |

Vergleichsbeispiel 1 beschreibt die Herstellung von mechanischen Retentionen mittels Retentionsperlen, was ein übliches Verfahren des Standes der Technik ist. Hierbei ist kein chemischer Verbund zwischen Metall und Kunststoff zu erzielen, sondern es handelt sich um eine rein mechanische Verankerung, die zwar nachweislich gute Haftergebnisse liefert, aber aufgrund des mangelnden chemischen Verbundes zu Randspalten zwischen Kunststoff und Metall führt und ausserdem einen erhöhten Metallauftrag und hiermit eine Beeinflussung der einzustellenden Farbe der Kunststoffverblendung bewirkt.

Vergleichsbeispiel 2 beschreibt einen Versuch, bei dem lediglich mit handelsüblichem Korund sandgestrahlt wurde.

Vergleichsbeispiel 3 entspricht Beispiel 2; lediglich wurde die behandelte Oberfläche nachher silanisiert.

Vergleichsbeispiel 4 entspricht Beispiel 3; lediglich wurde hier als Strahlmittel Quarz mit einer mittleren Korngrösse von 25 »m benutzt.

### Diskussion der Vergleichsbeispiele 1 bis 4

Lediglich mit mechanischen Retentionen lassen sich genügende Haftfestigkeiten erreichen (aus klinischen Versuchen ist bekannt, dass Haftwerte > 6,7 MPa den üblichen Kaubelastungen im Mund standhalten, siehe z.B. M. Rimpler, R. Hallbach-Moritz, G. Geibel und M. Pepping, Deutsche Zahnärztliche Zeitung, Bd. 37, Seite 321-324, 1982). Das alleinige Sandstrahlen mit unsilanisiertem Aluminiumoxid oder Quarz ergibt auch nach Nachbehandlung mit Silanisierlösungen keine befriedigenden Haftergebnisse.

### Beispiele 5 bis 11 (silanisierte Strahlmittel)

Probeplättchen aus Degulor M (Fa. Degussa) wurden 30 sec. mit dem SiO₂-haltigen Strahlmittel bei 4 bar abgestrahlt (Sandstrahlgerät FG-32 Sandmaster).

| Nr. | Strahlmittel | Nachbehandlung (Silanisierung) | Haftfestigkeit (MPa) Durchschnitt aus 5 Proben |
|---|---|---|---|
| 5 | Quarz 25 »m⁽⁴⁾ 1 % sil.⁽¹⁾ | Silanisierlösung A | 10,5 (erfindungsgemäss) |
| 6 | Quarz 25 »m⁽⁴⁾ 1 % sil.⁽¹⁾ | Silanisierlösung B⁽²⁾ | 14,4 (erfindungsgemäss) |
| 7 | Quarz 25 »m⁽⁴⁾ 3 % sil.⁽¹⁾ | Silanisierlösung A | 10,2 (erfindungsgemäss) |
| 8 | Duran®-Glas⁽³⁾ ca. 70 % SiO₂ 1 % sil.⁽¹⁾ | Silanisierlösung A | 8,9 (erfindungsgemäss |
| 9 | Duran®-Glas⁽³⁾ 3 % sil.⁽¹⁾ | Silanisierlösung A | 11,3 (erfindungsgemäss) |
| 10 | Al₂O₃ 110 »m⁽⁴⁾ 3 % sil.⁽¹⁾ | Silanisierlösung A | 4,26 (Vergleich) |
| 11 | Al₂O₃ 26 »m⁽⁴⁾ 3 % sil.⁽¹⁾ | Silanisierlösung A | 4,27 (Vergleich) |

| | | | |
|---|---|---|---|
| (1) nach üblicher Silanisierungsmethode mit γ-Methacroyloxypropyltrimethoxysilan | | | |
| (2) wie Silanisierlösung A, jedoch statt γ-Methacroyloxypropyltrimethoxysilan Divinyl-tetramethyl-disilizan | | | |
| (3) Fa. Schott, mittlere Korngrösse ca. 20 »m | | | |
| (4) mittlere Korngrösse | | | |

### Diskussion der Beispiele 5 - 11

Die Beispiele 5 bis 11 zeigen, dass ausreichende Haftfestigkeiten nur dann erreicht werden, wenn das silanisierte homogene Strahlmittel eine genügende Menge an Siliciumdioxid enthält. Bei den Versuchen 5 bis 9 (erfindungsgemäss) erfolgte der Riss immer in der Kunststoffschicht (Adhäsionsfähigkeit grösser als Kohäsionsfähigkeit).

### Beispiele 12 und 13 (unsilanisiertes Strahlmittel, welches pyrogene Kieselsäure enthält)

- Prüfplättchen:: Degulor M, Fa. Degussa
- Strahlmittel:: Al₂O₃, mittlere Korngrösse ca. 250 »m, enthaltend pyrogene Kieselsäure mit mittlerer Korngröse von ca. 0,04 »m (Aerosil ® OX50-Degussa)
- Strahldauer:: 10 sec. (5 bar)
- Nachbehandlung:: Silanisierlösung A

| Nr. | Anteil an pyrogener Kieselsäure (Gew.-%) | Haftfestigkeit (MPa) Durchschnitt aus 5 Proben |
|---|---|---|
| 12 | 5 | 12,8 |
| 13 | 20 | 11,8 |

### Diskussion der Beispiele 12 und 13

Die Versuche zeigen, dass geringe Anteile unsilanisierter pyrogener Kieselsäure zu einem handelsüblichen Strahlmittel mit Silanisiernachbehandlung zu guten Haftergebnissen zwischen Kunststoff und Metall führen.

Der Riss erfolgte in jedem Fall innerhalb der Kunststoffschicht, d.h. dass die Adhäsionsfestigkeit der Klebeverbindung grösser ist als die Kohäsionsfestigkeit des Kunststoffs.

### Beispiele 14 - 18 (verschiedene Metalle)

- Strahlmittel:: Al₂O₃, mittlere Korngrösse ca. 250 »m, enthaltend 5 Gew.-% pyrogene Kieselsäure mit mittlerer Korngröse von ca. 0,04 »m (Aerosil® OX50)
- Silanisierungsgrad der Kieselsäure:: 3 %
- Strahldauer:: 10 sec. bei 5 bar
- Nachbehandlung:: Silanisierlösung A

| Nr. | verwendetes Metall | Haftfestigkeit (MPa) Durchschnitt aus 5 Proben |
|---|---|---|
| 14 | Degulor M⁽¹⁾ | 12,8 |
| 15 | Stabilor G⁽²⁾ | 12,8 |
| 16 | Wiron 88⁽³⁾ | 13,2 |
| 17 | Wironit⁽⁴⁾ | 16,7 |
| 18 | Palliag⁽⁵⁾ | 10,1 |

| | | |
|---|---|---|
| (1) Hochgoldhaltige Legierung, Fa. Degussa | | |
| (2) Edelmetallreduzierte Legierung, Fa. Degussa | | |
| (3) Chrom-Nickel-Legierung, Fa. Bego | | |
| (4) Chrom-Cobalt-Legierung, Fa. Bego | | |
| (5) Palladium-Silber-Legierung, Fa. Degussa | | |

### Diskussion der Beispiele 14 - 18

Die Ergebnisse zeigen eindrucksvoll, dass mit dem erfindungsgemässen Verfahren alle auf dem Dentalgebiet handelsüblichen Metalle von hochgoldhaltigen Legierungen bis zu Nicht-Edelmetallegierungen verwendet werden können.

### Beispiele 19 - 21 (erfindungsgemäss verwendete Strahlmittel ohne Silanisiernachbehandlung)

- Prüfplättchen:: Degulor M, Fa. Degussa
- Strahldauer:: 10 sec. bei 5 bar
- Nachbehandlung:: keine

| Nr. | pyrogene Kieselsäure mit mittlerer Korngrösse von ca. 0,04 »m (Gew.-%) | Strahlmittel Al₂O₃ mittlere Korngrösse | Haftfestigkeit (MPa) Durchschnitt aus 5 Proben |
|---|---|---|---|
| 19 | 5 % 3 % sil. | ca. 250 »m | 8,2 |
| 20 | 5 % unsil. | ca. 250 »m | 11,1 |
| 21 | 1 % unsil. | ca. 110 »m | 10,9 |

### Diskussion der Beispiele 19 - 21

Die Versuche zeigen, dass auch ohne eine Silanisiernachbehandlung gute Haftergebnisse zwischen Kunststoff und Metall erhalten werden.

### Beispiele 22 bis 25 mit verschiedenen Zusätzen

- Prüfplättchen:: Degulor M
- Strahlmittel:: Al₂O₃, mittlere Korngrösse ca. 250 »m, + Zusatz
- Strahldauer:: 10 sec. bei 5 bar
- Nachbehandlung:: Silanisierlösung A

| Nr. | Zusatz (Gew.-%) | Haftfestigkeit (MPa) Durchschnitt aus 5 Proben |
|---|---|---|
| 22 | 5 % pyrogene Kieselsäure mit mittlerer Korngrösse von ca. 0,008 »m (Aerosil 380, Degussa) (unsilanisiert) | 9,1 |
| 23 | 5 % pyrogene Kieselsäure mit mittlerer Korngrösse von ca. 0,008 »m (Aerosil 380, Degussa) (3 % silanisiert)⁽¹⁾ | 8,6 |
| 24 | 5 % Glaspulver mit mittlerer Korngrösse von 2 »m, enthält 25 Gew.-% Anteile mit Korngrösse < 1 »m (GM XO87, Fa. Schott, Landshut) (3 % silanisiert)⁽¹⁾ | 8,5 |
| 25 | 5 % Siliciumnitrid, enthält 60 Gew.-% Anteile < 1 »m (Fa. Toyo Soda, Japan) | 12,3 |

| | | |
|---|---|---|
| (1) mit γ-Methacryloxypropyl-trimethoxysilan | | |

### Diskussion der Beispiele 22 bis 25

Die Versuche zeigen, dass gute Haftergebnisse erhalten werden, wenn ausreichend siliciumhaltiges Material < 1 »m im Strahlmedium enthalten ist.
b) BEISPIELE FÜR METALL-METALL-VERKLEBUNGEN
   Messverfahren
   Die Haftfestigkeit der Verklebung wurde anhand eines Zug/Scherversuchs ermittelt. Formteile des zu prüfenden Metalls wurden entsprechend Figur 2 überlappend verklebt (Klebefläche 1 cm²).
   Die verklebten Proben wurden 6 Stunden einem Temperaturwechselbad (15 ↔ 70°C) mit einminütigem Wechsel ausgesetzt. Anschliessend wurde mit Hilfe einer Universalprüfmaschine (Fa. Zwick Modell 1435) bei einem Vorschub von 1 mm/min. die Abrisskraft gemessen und daraus die Haftfestigkeit in MPa berechnet. Pro Versuch wurden 5 Verklebungen durchgeführt und daraus der Mittelwert berechnet.

### Beispiel 26

1. Säubern der Oberfläche durch 10 sec. Abstrahlen mit Al₂O₃ (110 »m mittlere Korngrösse).
2. 10 sec. Abstrahlen mit Al₂O₃ (110 »m mittlere Korngrösse) enthaltend 1 % pyrogene Kieselsäure mit mittlerer Korngrösse von ca. 0,04 »m (Aerosil OX 50) unsilanisiert bei 2,5 bar Strahldruck.
3. Auftragen einer frischbereiteten Silanisierlösung bestehend aus 1 Teil γ-Glycidoxypropyltrimethoxysilan und 15 Teilen einer Lösung aus 87 % Ethanol, 3 % Essigsäure und 10 % Wasser.
4. Nach Durchführung der Oberflächenbehandlung (Schritte 1 bis 3) wurde 2-Komponenten-Epoxyklebstoff (Uhu ® hart) 1 : 1 angemischt. Beide Klebflächen wurden bestrichen, mit 3 kp Druck fixiert und 20 Stunden bei Raumtemperatur ausgehärtet. Anschliessend wurde eine 10-minütige Nachhärtung bei 180°C durchgeführt.

### Beispiele 27 (Vergleich ohne Oberflächenbeschichtung)

Verklebung wie in Beispiel 26, jedoch besteht die Vorbehandlung lediglich aus Schritt 1 (10 sec. Abstrahlen mit Al₂O₃); die Schritte 2 und 3 entfallen.

### Beispiel 28

Verklebung wie in Beispiel 26, jedoch
- in Schritt 3:: anstelle γ-Glycidoxypropyltrimethoxysilan wurde γ-Methacroyloxypropyl-trimethoxysilan verwendet;
- in Schritt 4:: nach Durchführung der Oberflächenbehandlung erfolgte die Verklebung mit einem 2-Komponenten-Klebstoff auf Acrylatbasis (Nimetic® -Grip, Fa. ESPE). Die beiden Komponenten wurden im Verhältnis 1 : 1 vermischt, beiderseitig auf die Klebeflächen aufgetragen, mit 30 N Druck fixiert und 20 Stunden bei 23°C ausgehärtet.

### Beispiel 29 (Vergleich ohne Oberflächenbeschichtung)

Verklebung wie in Beispiel 28, jedoch besteht die Vorbehandlung lediglich aus Schritt 1 (Schritte 2 und 3 entfallen).

### Messergebnisse

| Beispiel | Klebstoff | Zugscherfestigkeit (MPa) |
|---|---|---|
| 26 (erfindungsgemäss) | Epoxyharz | 28,6 |
| 27 (Vergleich) | Epoxyharz | 13,6 |
| 28 (erfindungsgemäss) | Acrylat | 18,4 |
| 29 (Vergleich) | Acrylat | 13,0 |

### Diskussion der Beispiele 26 bis 29

Bei der Herstellung von Metall-Metall-Verklebungen erhält man mit dem erfindungsgemässen Verfahren deutlich höhere Zugscherfestigkeiten als wenn man die Oberfläche lediglich mit Al₂O₃ abstrahlt. Anhand der Versuche wurde demonstriert, dass man vorteilhafterweise beim erfindungsgemässen Verfahren einen Silanisierschritt 3 mit Silanen durchführt, deren funktionelle Gruppen eine Copolymerisation mit dem verwendeten Kleber ermöglichen. So kann man für das Verkleben mit Epoxyklebstoffen vorteilhafterweise Epoxy-funktionelle Silane einsetzen und zum Verkleben mit Acrylat-Klebstoffen Acrylat- oder Methacrylat-funktionelle Silane

### Beispiele 30 - 33 mit verschiedenen Zusätzen

- Prüfplättchen:: Degulor M, Mohs-Härte 6
- Strahlmittel:: Al₂O₃, mittlere Korngrösse ca. 250 »m + Zusatz
- Strahldauer:: 10 sec. bei 5 bar
- Nachbehandlung:: Silanisierlösung A

| Nr. | Zusatz (Gew.-%) | Mohs-Härte | Haftfestigkeit (MPa) Durchschnitt aus 5 Proben |
|---|---|---|---|
| 30 (erfindungsgemäss) | 2 % Zirkondioxid (pyrogen, mittlere Korngrösse ca. 0,04 »m) | 8 | 12,4 |
| 31 (erfindungsgemäss) | 2 % Aluminiumoxid (mittlere Korngrösse 0,04 -0,2 »m) | 9 | 18,3 |
| 32 (erfindungsgemäss) | 3 % Titandioxid (P 25, pyrogen, Fa. Degussa, mittlere Korngrösse 0,03 »m) | 6 - 7 | 12,8 |
| 33 (Vergleichsversuch) | 3 % Calciumfluorid gefällt (Fa. Merck, mittlere Korngrösse ca. 0,3 »m) | 4 | 5 |

### Ergebnis:

Die Ergebnisse zeigen, dass gute Haftwerte immer dann erreicht werden, wenn die Härte des erfindungsgemäss verwendeten Zusatzes grösser ist als die des Strahlmediums Degulor M mit einer Härte von 6 und die Korngrösse ausreichend klein ist. Bei jeder der beschriebenen Versuchsanordnungen ist nach Durchführung der Haftfestigkeitsmessung der Probekörper im Kunststoff, also im Opakermaterial gerissen
Beim Vergleichsversuch finden sich auf dem abgerissenen Metallplättchen keine Spuren des Kunststoffes (Opaker) mehr. Der Riss ist vollständig an der Grenzfläche Substrat/Kunststoff verlaufen.

### Beispiele 34 - 37, Haftung bei Kunststoff/Kunststoff-Verklebung

Die Versuchsanordnung ist analog den Beispielen 5 bis 11 mit dem Unterschied, dass als Substrat keine gegossenen Metallplättchen verwendet werden, sondern konfektionierte Kunststoffzähne auf vernetzter PMMA-Basis (Biodent, Firma DeTrey/Dentsply). Die Haftfläche der Kunststoffzähne wird plan geschliffen und (im Gegensatz zu den Beispielen 5 bis 11) zur Reinigung 5 Sekunden mit Aluminiumoxid < 110 »m abgestrahlt.

Die erfindungsgemässe Behandlung wird durchgeführt mit Aluminiumoxid < 110 »m plus die in der Tabelle angegebenen Mengen an erfindungsgemässem Zusatz. Anschliessend wird mit Silanisierungslösung B bestrichen, das runde sich nach oben konisch öffnende Metallhütchen (4) aufgesetzt und direkt schichtweise mit lichthärtendem Verblend Kunststoff (5) (Visio® -Gem, Firma ESPE) aufgefüllt und ausgehärtet.

| Nr. | Strahlmittelzusatz (Gew.-%) | Mohshärte | Mohshärte des Substrats (Biodentzahn) | Haftfestigkeit (MPa) |
|---|---|---|---|---|
| 34 (erfindungsgemäss) | 2 Gew.-% SnO₂ (mittlere Korngrösse ca. 0,8 »m) | 6-7 | 3 | 11 |
| 35 (erfindungsgemäss | 2 Gew.-% Calciumfluorid (Merck, mittlere Korngrösse ca. 0,3 »m) | 4 | 3 | 12,5 |
| 36 (erfindungsgemäss) | 1 Gew.-% pyrogene Kieselsäure mit mittlerer Korngrösse von ca. 0,04 »m | 6-7 | 3 | 11,5 |
| 37 (Vergleichsversuch) | - | | 3 | 3,5 |

### Ergebnis:

Die Haftwerte steigen bei erfindungsgemässer Vorbehandlung der Kunststoffoberflächen auf den 3- bis 4-fachen Wert an.

### Beispiele 38 - 39, Keramik/Kunststoff-Verklebung

Die Versuchsanordnung entspricht den Beispielen 34 - 37 mit dem Unterschied, dass als Substrat Prüfplättchen aus Dentalkeramik (VMK-Keramik, Firma Vita) verwendet werden.

| Nr. | Strahlmittelzusatz (Gew.-%) | Mohshärte | Mohshärte des Substrats (Biodentzahn) | Haftfestigkeit (MPa) |
|---|---|---|---|---|
| 38 (erfindungsgemäss) | 1 Gew.-% pyrogene Kieselsäure mit mittlerer Korngrösse von ca. 0,04 »m | 6-7 | 6 | 13,5 |
| 39 (Vergleichsversuch) | - | - | 6 | 7,5 |

### Ergebnis:

Mit dem erfindungsgemässen Zusatz lässt sich eine Steigerung der Haftfestigkeit um ca. 50 % erreichen, die Bruchbilder zeigen beim erfindungsgemässen Zusatz einen reinen kohäsiven Bruch, während bei dem Vergleichsversuch an ca. 50 % der Bruchfläche ein reiner Adhäsivbruch vorliegt.

### Beispiele 40 - 43

Zur Verklebung zweier Kunststoffplättchen aus Polypropylen und Teflon werden jeweils Prüfkörper (25 mm Länge, 5 mm Breite, 2 mm Dicke) hergestellt und jeweils zwei solcher Prüfkörper den Oberflächenbehandlungen unterzogen. Anschliessend werden die Prüfkörper mit einer Lösung C (frischbereitete Lösung aus 1 Teil γ-Glycidoxypropyltrimethoxysilan und 15 Teilen einer Lösung aus 87 % Ethanol, 3 % Essigsäure und 10 % Wasser) bestrichen, trocken geblasen und aus der Hälfte der Länge mittels eines Zweikomponenten Epoxyharzes (E 32, Firma Delo) verklebt. Vor der Verklebung werden die Oberflächen mit Aluminiumoxid 110 »m für 10 Sekunden vorgestrahlt, bei einem Druck von 2 bar. Die Hälfte der Proben werden anschliessend erfindungsgemäss mit einem Zusatz von 1 Gew.-% pyrogener Kieselsäure mit mittlerer Korngrösse von ca. 0,04 »m (OX 50, Firma Degussa) zum Aluminiumoxid für 10 Sekunden gestrahlt. Die Ergebnisse finden sich in der Tabelle.

| Nr. | Strahlmittelzusatz | Substrat | Haftfestigkeit (MPa) |
|---|---|---|---|
| 40 (erfindungsgemäss) | 1 Gew.-% pyrogene Kieselsäure mit mittlerer Korngrösse von ca. 0,04 »m | Polypropylen | 4 |
| 41 (Vergleichsversuch) | - | Polypropylen | 1,5 |
| 42 (erfindungsgemäss) | 1 Gew.-% pyrogene Kieselsäure mit mittlerer Korngrösse von ca. 0,04 »m | Teflon | 3 |
| 43 (Vergleichsversuch) | - | Teflon | 1 |

### Ergebnis:

Mit der erfindungsgemässen Oberflächenbehandlung lässt sich eine doppelt bis dreifach so hohe Haftfestigkeit erzielen.

## Patentansprüche

1. Verfahren zur Vorbereitung einer Substratoberfläche für die Verbindung mit Kunststoff, bei welchem man auf die Substratoberfläche eine Schicht aufbringt, dadurch gekennzeichnet, dass diese Schicht durch Sandstrahlen mit einem Mittel aufgebracht wird, das, bezogen auf das Gewicht des gesamten Sandstrahlmittels, aus
(A) 0,01 bis 90 Gew.-% gegebenenfalls silanisiertem Material mit einer Korngrösse < 5 »m und einer grösseren Härte als die der Substratoberfläche und/oder
(B) 20 bis 100 Gew.-% silanisiertem, siliciumhaltigem Material mit einer mittleren Korngrösse von 2 bis 200 »m und zum
(C) Rest aus einem Sandstrahlmittel mit einer mittleren Korngrösse > 5 »m
besteht, und gegebenenfalls die so erhaltene Haftvermittlerschicht anschliessend silanisiert wird.

2. Verfahren nach Anspruch 1, dadurch gekennzeichnet, dass man als Substratoberfläche eine Metalloberfläche verwendet.

3. Verfahren nch Anspruch 1 oder 2, dadurch gekennzeichnet, dass man als (A) 0,01 bis 50 Gew.-% gegebenenfalls silanisiertes, siliciumhaltiges Material verwendet.

4. Verfahren nach einem der Ansprüche 1 bis 3, dadurch gekennzeichnet, dass man als (A) Quarz, Quarzglas, Silikatglas mit mindestens 10 Gew.-% Silicium, pyrogene Kieselsäure, Siliciumcarbid und/oder Siliciumnitrid verwendet.

5. Verfahren nach Anspruch 1 oder 2, dadurch gekennzeichnet, dass man als (A) Aluminiumoxid, Titandioxid und/oder Zirkondioxid verwendet.

6. Verfahren nach einem der Ansprüche 1 bis 5, dadurch gekennzeichnet, dass man (A) mit einer Korngrösse < 1 »m, besonders < 0,1 »m, verwendet.

7. Verfahren nach einem der Ansprüche 1 bis 4, dadurch gekennzeichnet, dass man als (A) pyrogene Kieselsäure mit einer mittleren Korngrösse von 0,001 bis 0,05 »m verwendet.

8. Verfahren nach einem der Ansprüche 1 bis 7, dadurch gekennzeichnet, dass man 0,1 bis 30 Gew.-% (A) verwendet.

9. Verfahren nach einem der Ansprüche 1 bis 8, dadurch gekennzeichnet, dass man als (B) silanisierten Quarz, silanisiertes Quarzglas, silanisiertes Silikatglas mit mindestens 10 Gew.-% Silicium, silanisiertes keramisches Material mit mindestens 10 Gew.-% Silicium, silanisiertes Siliciumnitrid und/oder silanisiertes Siliciumcarbid verwendet.

10. Verfahren nach Anspruch 9, dadurch gekennzeichnet, dass man (B) mit einer mittleren Korngrösse von > 5 bis 100 »m verwendet.

11. Verfahren nach Anspruch 9 oder 10, dadurch gekennzeichnet, dass man 50 bis 100 Gew.-% (B) verwendet.

12. Verfahren nach den Ansprüchen 1 bis 11, dadurch gekennzeichnet, dass man auf die vorbereitete Substratoberfläche anschliessend eine Klebstoffschicht aufträgt.

13. Verwendung von gegebenenfalls silanisiertem Material mit einer Korngrösse < 5 »m und/oder von silanisiertem, siliciumhaltigem Material mit einer mittleren Korngrösse von 2 bis 200 »m zur Herstellung eines Sandstrahlmittels, mittels welchem Substratoberflächen für die Verbindung mit Kunststoff dadurch vorbereitet werden, daß mittels des Sandstrahlmittels auf die Substratoberfläche eine Schicht aufgebracht wird, wobei das gegebenenfalls silanisierte Material eine größere Härte aufweist als die zu bearbeitende Substratoberfläche.

14. Verfahren zum Verkleben, Beschichten oder Verblenden von Substratoberflächen mit Kleb-, Beschichtungs- oder Verblendmassen aus Kunststoff, bei welchem man auf die Substratoberfläche eine Schicht aufbringt, dadurch gekennzeichnet, daß diese Schicht durch Sandstrahlen mit einem Mittel aufgebracht wird, das, bezogen auf das Gewicht des gesamten Sandstrahlmittels aus
(A) 0,01 bis 90 Gew.-% gegebenenfalls silanisiertem Material mit einer Korngröße < 5 »m und einer größeren Härte als die der Substratoberfläche und/oder
(B) 20 bis 100 Gew.-% silanisiertem, siliciumhaltigem Material mit einer mittleren Korngröße von 2 bis 200 »m und zum
(C) Rest aus einem Sandstrahlmittel mit einer mittleren Korngröße > 5 »m
besteht, wobei die so erhaltene Haftvermittlerschicht anschließend gegebenenfalls silanisiert wird, und daß die Haftvermittlerschicht mit Kleb-, Beschichtungs- oder Verblendmassen aus Kunststoff verklebt, beschichtet oder verblendet wird.

## Claims

1. Process for preparing a substrate surface for bonding with a synthetic resin and with which a layer is applied on the substrate surface, characterized by that layer being applied by sand blasting with a composition which, based on the weight of the entire sand blasting composition, consists of
(A) 0.01 to 90% by weight of an optionally silanized material having a particle size < 5 »m and having a hardness exceeding that of the substrate surface and/or
(B) 20 to 100% by weight of silanized silicon-containing material having an average particle size of 2 to 200 »m and the
(C) remainder of a sand blasting composition having an average particle size > 5 »m,
and the thus obtained adhesion promoter layer thereafter optionally being silanized.

2. Process according to claim 1, characterized by using a metal surface as substrate surface.

3. Process according to claims 1 or 2, characterized by using as (A) 0.01 to 50% by weight of optionally silanized silicon-containing material.

4. Process according to one of claims 1 to 3, characterized by using as (A) quartz, quartz glass, silicate glass having at least 10% by weight of silicon, pyrogenic silicic acid, silicon carbide and/or silicon nitride.

5. Process according to claims 1 or 2, characterized by using as (A) alumina, titania and/or zirconia.

6. Process according to claims 1 to 5, characterized by using (A) having a particle size of < 1 »m, especially < 0.1 »m.

7. Process according to one of claims 1 to 4, characterized by using as (A) pyrogenic silicic acid having an average particle size of 0.001 to 0.05 »m.

8. Process according to one of claims 1 to 7, characterized by using 0.1 to 30% by weight of (A).

9. Process according to one of claims 1 to 8, characterized by using silanized quartz, silanized quartz glass, silanized silicate glass having at least 10% by weight of silicon, silanized ceramic material having at least 10% by weight of silicon, silanized silicon nitride and/or silanized silicon carbide.

10. Process according to claim 9, characterized by using (B) having an average particle size of > 5 to 100 »m.

11. Process according to claims 9 or 10, characterized by using 50 to 100% by weight of (B).

12. Process according to claims 1 to 11, characterized by subsequently applying an adhesive layer on the prepared substrate surface.

13. Use of optionally silanized material having a particle size < 5 »m and/or of silanized silicon-containing material having an average particle size of 2 to 200 »m for producing a sand blasting composition by means of which substrate surfaces are prepared for bonding with synthetic resin by applying a layer on the substrate surface by means of the sand blasting composition, the optionally silanized material having a hardness exceeding that of the substrate surface to be treated.

14. Process for bonding, coating or facing substrate surfaces with bonding, coating or facing compositions of synthetic resin and with which a layer is applied on the substrate surface, characterized by that layer being applied by sand blasting with a composition which, based on the weight of the entire sand blasting composition, consists of
(A) 0.01 to 90% by weight of an optionally silanized material having a particle size < 5 »m and having a hardness exceeding that of the substrate surface and/or
(B) 20 to 100% by weight of silanized silicon-containing material having an average particle size of 2 to 200 »m and the
(C) remainder of a sand blasting composition having an average particle size > 5 »m,
the thus obtained adhesion promoter layer thereafter optionally being silanized, and by the adhesion promoter layer being bonded, coated or faced with bonding, coating or facing compositions of synthetic resin.

## Revendications

1. Procédé pour apprêter la surface d'un substrat en vue de son assemblage avec une matière plastique, dans lequel on applique une couche sur la surface du substrat, caractérisé en ce que cette couche est appliquée par sablage avec un agent qui est constitué, par rapport au poids de l'ensemble de l'agent de sablage, de :
(A) 0,01 à 90 % en poids d'un matériau éventuellement silané ayant une dimension de grains < 5 »m et une dureté supérieure à celle de la surface du substrat, et/ou
(B) 20 à 100 % d'un matériau silicié silané ayant une dimension moyenne de grains de 2 à 200 »m, et
(C) le reste étant formé d'un agent de sablage ayant une dimension moyenne de grains < 5 »m,
et ensuite, la couche d'adhérence ainsi obtenue est éventuellement silanée.

2. Procédé selon la revendication 1, caractérisé en ce qu'on utilise une surface métallique comme surface de substrat.

3. Procédé selon la revendication 1 ou 2, caractérisé en ce qu'on utilise, comme composant (A), de 0,01 à 50 % en poids d'un matériau silicié éventuellement silané.

4. Procédé selon l'une des revendications 1 à 3, caractérisé en ce qu'on utilise, comme composant (A), le quartz, le verre de quartz, un verre de silicates contenant au moins 10 % en poids de silicium, l'acide silicique pyrogène, le carbure de silicium et/ou le nitrure de silicium.

5. Procédé selon la revendication 1 ou 2, caractérisé en ce qu'on utilise, comme composant (A), l'oxyde d'aluminium, le dioxyde de titane et/ou le dioxyde de zirconium.

6. Procédé selon l'une des revendications 1 à 5, caractérisé en ce qu'on utilise un composant (A) ayant une dimension de grains < 1 »m, en particulier < 0,1 »m.

7. Procédé selon l'une des revendications 1 à 4, caractérisé en ce qu'on utilise, comme composant (A), de l'acide silicique pyrogène ayant une dimension moyenne de grains de 0,001 à 0,05 »m.

8. Procédé selon l'une des revendications 1 à 7, caractérisé en ce qu'on utilise de 0,1 à 30 % en poids de composant (A).

9. Procédé selon l'une des revendications 1 à 8, caractérisé en ce qu'on utilise, comme composant (B), du quartz silané, du verre de quartz silané, du verre de silicates silané contenant au moins 10 % en poids de silicium, une matière céramique silanée contenant au moins 10 % en poids de silicium, du nitrure de silicium silané et/ou du carbure de silicium silané.

10. Procédé selon la revendication 9, caractérisé en ce qu'on utilise un composant (B) ayant une dimension moyenne de grains > 5 à 100 »m.

11. Procédé selon la revendication 9 ou 10, caractérisé en ce qu'on utilise de 50 à 100 % en poids de composant (B).

12. Procédé selon les revendications 1 à 11, caractérisé en ce qu'on applique ensuite une couche de colle sur la surface apprêtée du substrat.

13. Utilisation d'un matériau éventuellement silané ayant une dimension de grains < 5 »m et/ou d'un matériau silicié silané ayant une dimension moyenne de grains de 2 à 200 »m, pour la préparation d'un agent de sablage au moyen duquel on apprête des surfaces des substrats pour leur assemblage avec une matière plastique, d'une manière telle qu'une couche est appliquée sur la surface du substrat au moyen de l'agent de sablage, le matériau éventuellement silané ayant une dureté supérieure à celle de la surface du substrat à traiter.

14. Procédé de collage, d'enduction ou de revêtement de surfaces de substrats avec des masses de collage, d'enduction ou de revêtement à base de matière plastique, dans lequel on applique une couche sur la surface du substrat, caractérisé en ce que cette couche est appliquée par sablage avec un agent qui est constitué, par rapport au poids de l'ensemble de l'agent de sablage, de :
(A) 0,01 à 90 % en poids d'un matériau éventuellement silané ayant une dimension de grains < 5 »m et une dureté supérieure à celle de la surface du substrat, et/ou
(B) 20 à 100 % d'un matériau silicié silané ayant une dimension moyenne de grains de 2 à 200 »m, et
(C) le reste étant formé d'un agent de sablage ayant une dimension moyenne de grains < 5 »m,
la couche d'adhérence ainsi obtenue étant ensuite éventuellement silanée, et en ce que la couche d'adhérence est collée, enduite ou revêtue avec des masses de collage, d'enduction ou de revêtement à base de matière plastique.
